# EUROPÄISCHE PATENTSCHRIFT

(11) **EP 0 617 972 B1**
(45) Veröffentlichungstag und Bekanntmachung des Hinweises auf die Patenterteilung: **09.07.2003**
(21) Anmeldenummer: 94104381.2
(22) Anmeldetag: 19.03.1994
(51) Int. Cl.: A61L 15/58, A61L 15/44

(54) **Dermales therapeutisches System aus einer schmelzfähigen Poly(meth)-acrylat-Mischung**
Dermal therapeutic system based on a mixture of fusible polymethacrylates
Système thérapeutique dermique à base d'un mélange de polyméthacrylates fusibles

(30) Priorität: 27.03.1993 DE 4310012
(43) Veröffentlichungstag der Anmeldung: 05.10.1994
(73) Patentinhaber: Röhm GmbH & Co. KG, 64293 Darmstadt (DE)
(72) Erfinder: Lehmann, Klaus, Dr., D-64380 Rossdorf (DE); Petereit, Hans-Ulrich, D-64291 Darmstadt (DE); Assmus, Manfred, D-64404 Bickenbach (DE)

(56) Entgegenhaltungen:
- EP-A- 0 261 402
- EP-A- 0 415 055
- WO-A-85/03878
- DE-A- 3 823 070
- LIN SHAN-YANG ET AL: "The effect of plasticizers on compatibility, mechanical properties, and adhesion strength of drug-free Eudragit E films" PHARMACEUTICAL RESEARCH, Bd. 8, Nr. 9, 1991, Seiten 1137-1143, XP002107579

## Beschreibung

### Gebiet der Erfindung

Die Erfindung betrifft zum Aufbringen auf die Haut vorgesehene, dermal und transdermal wirkende Therapiesysteme, bei denen die Abgabe des Wirkstoffs an die Haut durch Polymere gesteuert wird.

### Stand der Technik

Für eine geregelte und kontinuierliche Freigabe von Wirkstoffen an die Haut oder durch die Haut werden üblicherweise Pflaster verwendet, bei denen eine Klebemasse mit einer Pharmaka-haltigen Schicht und einer Trägerfolie oder einem Stoffgewebe, zu einer mehrschichtigen Zubereitung formuliert sind.

Als Klebemassen dienen zur Befestigung von flächigen flexiblen Substraten auf der Haut druckempfindliche Hauthaftkleber. Mit Hauthaftklebern lassen sich nicht nur wirkstoffhaltige Substrate, wie Wundpflaster oder medizinische Haftpflaster fest und dauerelastisch an der Haut befestigen. Sie eignen sich auch zum Aufbau von wirkstoffhaltigen, örtlich oder transdermal wirkenden, auf der Haut haftenden Schichten, d.h. von Arzneimitteln mit topischem oder systemischem pharmazeutischem Wirkstoff. Vorteilhaft läßt sich diese Arzneiform als Depotpräparat für die verzögerte Freigabe des Wirkstoffes und damit auch länger anhaltenden Wirkung einsetzen.

Im allgemeinen werden solche dermalen oder transdermalen Systeme aus mehreren Schichten aufgebaut: Trägerfolie, Depotschicht mit Arzneimittel, Diffusion kontrollierende Schicht, auf der Haut haftende Schicht und Abdeckfolie, die vor der Anwendung abgezogen wird. Es besteht jedoch zur Vereinfachung der Herstellung oft der Wunsch, weniger Schichten aufzubringen und die Funktion mehrerer Schichten in einer zu vereinigen, im einfachsten Fall nur eine Schicht, die auf der Haut klebt aber auch den Wirkstoff enthält. Trägerfolie und Abdeckfolie haben dann nur die Funktion einer Verpackung.

Für die Herstellung von Hauthaftklebern dienen klebrige Polymere natürlicher oder synthetischer Herkunft. Als auf der Haut fixierbare synthetische Polymere sind filmbildende Poly(meth)acrylate, mit ihren durch die Herstellung möglichen, großen Eigenschaftsvarietäten schon seit vielen Jahren als Hauthaftkleber und für transdermale therapeutische Systeme bekannt.

In der DE-OS 36 12 305 ist ein pharmazeutisches Mittel zur lokalen und reizarmen Therapie der Psoriasis aus an sich bekannten antipsoriatischen Wirkstoffen und aus filmbildenden Polymeren in Lösung oder in Dispersion beschrieben, aus dem sich nach Auftragung auf die befallene Hautstelle, der therapeutisch wirksame Film bildet.

Aus der EP-A 0 394 956 sind anionische (Meth)acrylat-Copolymerisate und aus der EP-A 0 415 055 sind kationische (Meth)acrylat-Copolymerisate bekannt, die sich in Form ihrer wassergelösten Salze in gleicher Weise zur Bildung von Hauthaftklebern für Wundpflaster und für die Bildung eines transdermal wirkenden, zum Aufbringen auf die Haut vorgesehenen Arzneimittels eignen.

Die deutsche Patentschrift DE-C 32 08 853 beansprucht eine pharmazeutische Verbundzubereitung für die Anhaftung auf der Haut durch eine selbstklebende Masse, die auch die pharmazeutische Wirksubstanz in einer höheren Konzentration als es deren Löslichkeit in der Klebesubstanz entspricht, enthält, bei der diese Mischung auf einem Polymerfilm aufgebracht ist, den die Wirksubstanz durchwandern kann. Die Anforderungen an die Klebeeigenschaften, Verträglichkeit, Löslichkeit und Freigabefähigkeit werden danach am besten von Acryl-Copolymeren erfüllt. Acryl-Copolymere, das sind bevorzugt Copolymere mit wenigstens 50 Gew.-% an Alkylacrylat oder Alkylmethacrylat mit durchschnittlich wenigstens 4 Kohlenstoffatomen im Alkylrest. Sie werden als Lösung mit dem Wirkstoff zum transdermalen therapeutischen System formuliert.

EP-A 0 261 402 beschreibt ein transdermales Therapiesystem, seine Verwendung und Verfahren zu seiner Herstellung. Gemäß EP-A 0 261 402 kann eine Haftklebermasse aus einer Mischung eines selbstvernetzenden Acrylatpolymeren (Durota® 280-2416, National Starch & Chemical B.V.) und einem Copolymerisat aus (50 %) Dimethylaminoethylmethacrylat und (50 %) neutralem Methacrylsäureester (EUDRAGIT® E100) hergestellt werden.

Weiterhin ist in dieser Mischung kein pharmazeutischer Wirkstoff enthalten. Der Wirkstoff Nicotin wird nachträglich in einen Vliesstoff in Form einer Lösung aus EUDRAGIT® E100 aufgebracht, liegt also nicht in einer Polymermischung vor.

DE 38 23 070 A1 beschreibt selbstklebende Heftpflaster zur Abgabe von Arzneistoffen an die Haut. Gemäß DE 38 23 070 A1 kann die selbstklebende Schicht der Polymermatrix 2 wird ein oder mehrere quellbare polymere Matrixbildner umfassen. Als Matrixbildner werden eine Reihe verschiedener Polymere u. a auch Polyisobutylmethacrylat und Polyhydroxyethylmethacrylat genannt.

Letzteres **umfaßt** zwar hydrophile funktionelle Gruppen, **ist** jedoch ein Homopolymerisat und kein Copolymerisat.

### Problem und Lösung

### Poly(meth)acrylate sind wegen ihrer guten

Hautverträglichkeit ausgezeichnete Hilfsstoffe für dermale Arzneiformen. Die Anwendung in Form organischer Lösungen ist wegen der Feuergefährlichkeit und Toxizität der Lösungsmittel und erheblichem technischem und organisatorischem Aufwand zum Schutz des Bedienungspersonals und Entsorgung der Lösungsmittel verbunden. Wäßrige Dispersionen sind gefahrlos zu handhaben, doch erfordert das Trocknen hohen Energieaufwand, sorgsam gesteuerte Trocknungsprozeduren und damit ebenfalls komplizierte technische Einrichtungen. Die einsetzbaren Dispersionen sind außerdem anfällig gegen mikrobiologisches Wachstum, sind nur begrenzt lagerstabil, können bei Wirkstoffzusatz koagulieren und enthalten Emulgatoren, die die Funktion der Arzneiform und deren Lagerstabilität, sowie die Verträglichkeit auf der Haut, erheblich beeinträchtigen können.

Es wurde nun gefunden, daß man durch ein Aufschmelzen und Ausstreichen (Hot-melt-Verfahren) von ausgewählten Mischungen von Poly(meth)acrylaten in einfacher Weise Arzneistoffe zur Abgabe an die Haut oder Schleimhaut formulieren kann. Durch Abstimmung der Polymerkomponenten mit den Wirkstoffen und weiteren Hilfsstoffen wie Weichmacher, penetrationsfördernde Substanzen, u.a., ist es nun möglich, die Wirkstoffabgabe an die Haut zu steuern und therapiegerechte Freigabeprofile zu erzielen.

Die Polymer-Mischungskomponenten werden nach zwei wesentlichen Gesichtspunkten ausgewählt:
1. Beeinflussung der Wirkstoffabgabe durch (Meth)acryl-Polymere, die funktionelle Gruppen enthalten
2. Beeinflussung des Schmelz- und Fließverhaltens durch Poly(meth)acrylate, die keine oder nur unerhebliche Mengen an funktionellen Gruppen enthalten. Weichmacher werden ggf. zur Herabsetzung der Schmelztemperatur und Verminderung der Schmelzviskosität zusätzlich benötigt.

Die Erfindung betrifft ein:

Schichtförmiges dermales therapeutisches System mit verzögerter Wirkstoffabgabe mit mindestens einem pharmazeutischen Wirkstoff, dadurch gekennzeichnet, daß einer oder mehrere Schichten aus Mischungen von Poly(meth)acrylaten aufgebaut und aus einer Schmelze hergestellt werden und die Mischungskomponente 1 aus (Meth)acryl-Polymeren besteht, die funktionelle Gruppen enthalten, die Mischungskomponente 2 das Fließverhalten reguliert und nur unerhebliche Mengen an funktionellen Gruppen enthält.

Die polymeren Mischungskomponenten sind einmal filmbildende Polymere auf (Meth)acrylat-Basis, die funktionelle Gruppen enthalten, und durch Wechselwirkung mit dem System die Wirkstoffabgabe beeinflussen können, und daneben Polymere auf (Meth)acrylat-Basis, im wesentlichen aus C₁- bis C₁₂-Alkylestern der (Meth)acrylsäure, mit höchstens nur unerheblichen Mengen an Monomeren mit funktionellen Gruppen aufgebaut, und die das Schmelz- und Fließverhalten der polymeren Klebeschicht regulieren.

Die Molekulargewichte der erfindungsgemäß eingesetzten Polymere liegen zwischen 10.000 und 2.000.000 g/Mol.

Zur Herabsetzung der Schmelztemperatur und Verminderung der Schmelzviskosität der Polymermischung können gegebenenfalls noch niedermolekulare Weichmacher zugesetzt werden.

Auch ist der Zusatz pharmazeutischer Hilfsstoffe wie penetrationsfördernde oder -hemmende Substanzen, Konservierungsstoffe, Farbstoffe u.a. möglich.

### Ausführung der Erfindung

(1) Zur Herstellung der erfindungsgemäßen wirkstoffhaltigen Hauthaftkleber werden als funktionelle Gruppen enthaltende Poly(meth)acrylate, beispielsweise die für das Überziehen von Arzneimitteln als ®Eudragit bekannten Acrylharze eingesetzt. Diese Polymeren sind Copolymerisate von Methacrylestern und Acrylestern und gegebenenfalls auch weiteren Vinylmonomeren, mit Monomeren mit funktionellen Gruppen, wodurch die Polymeren kationischen, anionischen oder hydrophilen Charakter haben. Die Zusammensetzung des funktionellen Polymerisats sollte so sein, daß der daraus gebildete Film zwar trocken und hart, aber nicht spröde ist. Dieses Eigenschaftsbild zeigen Polymerisatfilme, deren dynamische Glastemperatur (auch als Tₘₐₓ der T_{g(dym)}) nach DIN 53445 im Bereich von -10 bis 100 Grad C, vorzugsweise 10 bis 60 Grad C liegt. Comonomere, die den funktionellen Charakter der (Meth)acrylat-Polymeren erbringen, sind beispielsweise Acrylsäure, Methacrylsäure, Maleinsäure für anionische Polymere, oder N-Dimethylamino-ethylmethacrylat oder - acrylat, N-[3-(Dimethylamino)-2,2-dimethylpropyl]-methacrylamid für kationische Polymere oder 2-Hydroxyethyl(meth)acrylat, 2-Hydroxypropyl(meth)acrylat und Trimethylammoniumethyl-(meth)acrylatchlorid für Polymere hydrophilen Charakters.
   Die Herstellung der erfindungsgemäß zu verwendenden funktionellen Poly(meth)acrylate geschieht in bekannter Weise wie üblich, durch Substanzpolymerisation, Lösungspolymerisation oder Emulsions(Dispersions)polymerisation. Für die Bereitung der Polymermischungen wird das Polymere als Feststoff in Form von Granulat bzw. Pulver mit Korngrößen von etwa 0,1 bis 0,5 mm verwendet, wozu es nach der Polymerisation, z.B. durch Granulierung von extrudiertem Substanzpolymerisat, wie es beispielsweise die kationischen Copolymerisate sind, oder durch Sprühtrocknung oder Gefriertrocknung von Emulsionspolymerisaten mit Teilchengrößen von ca. 50 bis 500 µ, wie z.B. die Säuregruppen-haltigen anionischen Poly(meth)acrylate, oder durch Eindampfen bzw. durch Ausfällen von Polymerlösungen, gewonnen wird.
   Beispiele für Poly(meth)acrylate mit funktionellen Gruppen sind:
   Copolymerisat mit ca. 50 Gew.-% Monomereinheiten mit tertiären Aminogruppen: ®Eudragit E 100
   Copolymerisat mit ca. 10 % quartären Ammoniumgruppen: ®Eudragit RS 100
   Copolymerisat mit ca. 50 Gew.-% Monomereinheiten mit Carbonsäuregruppen: ®Eudragit L 100-55 bzw. ®Eudragit L 100
   Copolymerisat mit ca. 30 Gew.-% Monomereinheiten mit Carbonsäuregruppen: ®Eudragit S 100
   Copolymerisate mit ca. 70 Gew.-% Monomereinheiten mit Carbonsäuregruppen: ®EUDISPERT
   Copolymerisate von Methylacrylat und Butylacrylat mit mehr als 10 Gew.-% Methacrylsäure
   Copolymerisate mit Hydroxyalkyl(meth)acrylat.
(2) Polymere ohne oder nur mit unerheblichen Mengen an funktionellen Gruppen, die in der erfindungsgemäßen Mischung das Schmelz- und Fließverhalten sowie die Klebrigkeit des neuen dermalen und transdermalen therapeutischen Systems ermöglichen, sind Poly(meth)acrylate, deren dynamische Glastemperaturen nach DIN 53445 im Bereich von -70 bis etwa 80 Grad C liegen. Besonders bevorzugt werden Poly(meth)acrylate deren Glastemperaturen im Bereich von -50 bis +70 Grad C, vorwiegend im Bereich von + 10 bis 70 Grad C liegen.
   Beispiele für solche Polymere sind:
   Copolymerisate aus Ethylacrylat und Methylmethacrylat, vorzugsweise mit mehr als 30 Gew.-% Ethylacrylat.
   Copolymerisate aus Ethylacrylat und Methylmethacrylat mit ca. 5 % Trimethylammonioethylmethacrylatchlorid.
   Copolymerisate von Methylacrylat und Methylmethacrylat. Copolymerisate von Methylmethacrylat mit Butyl(meth)acrylat und/oder 2-Ethylhexylmethacrylat.
(3) Dermale und transdermale Wirkstoffe, deren Abgabe aus dem auf der Haut aufgebrachten Therapiesystem durch die Polymerenkombination aus (1) und (2) gesteuert wird, müssen aufgrund ihrer physikalisch chemischen Eigenschaften in der Lage sein, am Wirkort, eine therapeutisch wirksame Konzentration aufzubauen. Die dermale Therapie erfordert oft das Eindringen von Arzneistoffmolekülen in tiefere Hautschichten. Für systemische Effekte ist die vollständige Passage der Haut, insbesondere des Stratum corneums notwendig, so daß der Arzneistoff nach Aufnahme in die Blutbahn im Körper verteilt wird. Für die erfindungsgemäße Verarbeitung sollten die Arzneistoffe eine ausreichende thermische Stabilität bei mindestens 50 Grad C aufweisen. Beispiele sind u.a. aus der DE-C 3 208 853 und umfangreicher Literatur bekannt.
   (I) Kortikosteroide, z.B. Hydrokortison, Prednisolon, Betamethason.
   (II) Analgetische, entzündungshemmende Mittel, beispielsweise Ibuprofen, Ketoprofen, Fentanyl.
   (III) Antihypertensive Mittel, z.B. Clonidin und Kallikrein.
   (IV) Antibiotika, z.B. Chloramphenicol, Neomycin.
   (V) Anästhetika, z.B. Lidokain.
   (VI) Fungizide, z.B. Klotrimazol.
   (VII) Vitamine und Derivate, z.B. Vitamin A-Säure, Chlolecalziferol.
   (VIII) Antiepileptika, z.B. Nitrazepam.
   (IX) Koronarvasodilatoren, z.B. Nitroglyzerin, Isosorbiddinitrat.
   (X) Antihistaminika, z.B. Diphenhydramin-hydrochlorid.
   (XI) Antipsoriatische Wirkstoffe, z.B. Dithranol.
   (XII) Sonstige Wirkstoffe wie Nicotin.
   (XIII) Hormone, z.B. Ostradiol, Testosteron.
   (XIV) Antiemetika, z.B. Scopolamin-Salze.

   In dem Therapiesystem können einzelne Wirkstoffe oder Kombination von Wirkstoffen enthalten sein. Ihre Menge in dem System beträgt 0,2 bis 50 Gew.-%, vorzugsweise 0,5 bis 20 Gew.-% bezogen auf die Polymermischung.
(4) Im Bedarfsfall können den Mischungen aus den Bestandteilen (1), (2) und (3) noch weitere Hilfsstoffe, beispielsweise Weichmacher, penetrationsfördernde Substanzen, z.B. Laurocapram, DMSO oder DMF, keratolytische Substanzen, wie z.B. Salicylsäure, Harnstoff oder auch Füllstoffe in Form feiner Pulver, z.B. aus SiO₂ oder CaCO₃, zugesetzt sein.

Trägerfolien können eine Dicke von vorzugsweise 50 - 500 µm besitzen. Sie bestehen aus üblichen Polymeren wie z.B. PP, PE, PVC, Polyestern, Polyurethan oder Polymethacrylaten, oder auch Aluminium. Die mechanische Stabilität sollte einerseits ein problemloses Beschichten ermöglichen und andererseits ausreichende Flexibilität beim Tragen auf der Haut gewährleisten. Die Folien sind oft hautfarben eingefärbt. Wenn nötig können Trägerfolien selbst schon einen schichtartigen Aufbau besitzen, z.B. Polymer/Aluminium-Kombinationen, um das Eindringen des Wirkstoffes in den Träger zu verhindern oder Primerbeschichtungen zur Verbesserung der Haftung der Wirkstoff/Polymermatrix auf dem Träger.

Trennfolien, die vor der Anwendung entfernt werden, bestehen grundsätzlich aus den gleichen Materialien.

Haftungsreduzierende Beschichtungen, wie z.B. Silikonverbindungen, ermöglichen ein einfaches Abtrennen.

Die im wesentlichen pulvrigen Bestandteile (1), (2), (3) und gegebenenfalls (4) werden in einem Kneter unter leichtem Druck gut vermischt und auf Temperaturen von 50 bis 200 Grad C, insbesondere auf 80 bis 120 Grad C erhitzt und die aufgeschmolzene Masse auf eine Trägerfolie aufgestrichen, wobei Dicken der wirkstoffhaltigen Polymerschicht von 10 bis 600 µm, vor allem von 50 bis 300 µm eingestellt werden. Als Träger werden wie üblich flächige Substrate, z.B. Folien aus Kunststoff oder Metall, besonders Aluminium, Textilien oder Papiere verwendet.

Das erfindungsgemäß hergestellte therapeutische System mit Poly(meth)acrylaten mit funktionellen Gruppen ermöglicht eine gesteuerte Abgabe des Wirkstoffs an die Haut und bei Wirkstoffen, die durch die Haut penetrieren, auch dessen Aufnahme durch die Haut. Die Kombination mit Poly(meth)acrylaten mit niedriger Glastemperatur ermöglicht schmelzfähige therapeutische Formulierungen auf einfache Weise herzustellen, die auch Eigenschaften eines druckempfindlichen Hauthaftklebers aufweisen können.

### BEISPIELE

### Beispiel 1

Eine Vormischung von Wirkstoff und Polymeren wurde hergestellt durch Verrühren von 100 mg Prednisolon, suspendiert in 5 ml Wasser, 5 g Polymerpulver, hergestellt aus gleichen Gew.-Teilen Methacrylsäure und Ethylacrylat und 5 ml einer 30 %igen wäßrigen Polymerdispersion, hergestellt aus Ethylacrylat und Methylmethacrylat im Gew.-Verhältnis 7 : 3. Die Vormischung wurde bei 40 Grad C zu einem Film getrocknet und bei 140 Grad C geschmolzen. Es entsteht eine weiche, knetbare Masse. Aus einem Filmstückchen von 4 x 4 cm und 0,6 mm Dicke, das etwa 32 mg Prednisolon enthält, wird der Wirkstoff in vitro in einer Freigabeapparatur nach USP (= United States Pharmacopeia USP XXII 1990, S. 1581, Transdermal Delivery Systems, General Drug Release Standards, Apparatus 3 Paddle over disk.) unter Verwendung eines Phosphatpuffers pH 6,8 wie folgt verzögert freigesetzt
nach Stunden
% Wirkstoff freigesetzt:
1 h 13 %
2 h 20 %
3 h 28 %
23 h 62 %
28 h 74 %

### Beispiel 2

Eine Vormischung wurde hergestellt durch Verrühren von 100 mg Prednisolon, suspendiert in 3 ml Wasser, 5 ml einer 30 %igen wäßrigen Polymerdispersion, hergestellt aus Ethylacrylat und Methylmethacrylat im Gew.-Verhältnis 7 : 3 und 5 ml einer 30 %igen Polymerdispersion hergestellt aus Methacrylsäure und Methylacrylat im Gew.-Verhältnis 8 : 2. Die Vormischung wurde bei 40 Grad C getrocknet und bei 120 Grad C geschmolzen. Aus einem Filmstückchen 4 x 4 cm und 0,3 mm Dicke wurde folgende Freigabe des Wirkstoffes nach der in Beispiel 1 angegebenen USP-Methode gefunden:
nach Stunden
% Wirkstoff freigesetzt:
1 h 25 %
2 h 38 %
3 h 45 %
5 h 55 %
24 h 66 %
48 h 72 %

### Beispiel 3

Es wurde wie in Beispiel 2 verfahren, jedoch hatte die Polymerdispersion hergestellt aus Methacrylsäure und Methylacrylat ein Gew.-Verhältnis dieser Monomeren von 9 : 1. Das Prednisolon wurde deutlich schneller und zwarwie folgt freigesetzt:
nach Stunden
% Wirkstoff freigesetzt
1 h 11 %
2 h 20 %
3 h 25 %
5 h 35 %
24 h 58 %
48 h 71 %

### Beispiel 4

Dieses Beispiel wurde wie Beispiel 2 durchgeführt, jedoch mit 100 mg Ketoprofen statt Prednisolon als Wirkstoff. Die Wirkstoffabgabe in vitro nach USP (wie in Beispiel 1) war wie folgt:
nach Stunden
mg Wirkstoff freigesetzt
1 h 8,3 mg
2 h 9,9 mg
4 h 10,9 mg
24 h 11,2 mg
48 h 11,8 mg

### Beispiel 5

Dieses wurde wie Beispiel 3 durchgeführt, jedoch ebenfalls mit 100 mg Ketoprofen statt Prednisolon. Die Wirkstoffabgabe in vitro nach USP (wie in Beispiel 1) war wie folgt:
nach Stunden
mg Wirkstoff freigesetzt
1 h 3,5 mg
2 h 5,4 mg
4 h 9,0 mg
24 h 14,4 mg
48 h 17,0 mg

### Beispiel 6

196 g ®EUDRAGIT E 100 und 196 g ®PLASTOID B werden mit 156 g Triethylcitrat in einem Kneter auf ca. 120 Grad C erhitzt und gleichmäßig durchmischt. Es entsteht eine klare, niedrigviskose Masse. Zu 450 g dieser Polymermischung werden 50 g Ketoprofen gegeben und gleichmäßig verteilt.

Diese Schmelze wird anschließend auf einer 50 µm starken Aluminiumfolie zu 1,9 mm dicken Schichten verteilt und bis zum Erstarren gekühlt.
Durch Ausstanzen erhält man runde Pflaster mit ca. 4,4 cm Durchmesser und ca. 285 mg Ketoprofen pro Einzeldosis. Die Bestimmung der in vitro Freigabe erfolgt nach USP XXII, S. 1582, Methode 4 (Zylinder) in Phosphatpuffer pH 6,8 und ergab folgende Werte (bezogen auf die anfängliche Wirkstoffbeladung):
1 Std.: 13,0 %
3 Std.: 24,5 %
6 Std.: 41,0 %
12 Std.: 59,8 %
18 Std.: 91,1 %
24 Std.: 96,8 %

### Beispiel 7

166,6 g ®EUDRAGIT E 100 und 166,6 g ®EUDRAGIT RS 100 werden mit 133,3 g Triethylcitrat in einem heizbaren Kneter bei ca. 120 Grad C gleichmäßig durchmischt. In 450 g der erhaltenen klaren, niedrigviskosen Schmelze verteilt man 50 g Ketoprofen gleichmäßig und streicht die Masse auf ca. 50 µm dicken Aluminiumfolien zu 1,9 mm dicken Schichten aus.

Nach dem Erstarren werden runde Pflaster mit 4,4 cm Durchmesser ausgestanzt. Jede Einzeldosis enthält 285 mg Ketoprofen. Die Bestimmung der Arzneistoffabgabe in vitro erfolgt nach USP XXII, S. 1582, Methode 4 (Zylinder) in Phosphatpuffer pH 6,8 und ergab folgende Werte (bezogen auf die anfängliche Wirkstoffbeladung):
1 Std.: 9,1 %
3 Std.: 16,7 %
6 Std.: 23,3 %
12 Std.: 35,9 %
18 Std.: 44,7 %
24 Std.: 51,2 %

### Beispiel 8

Eine Ketoprofen/Polymermischung gemäß Beispiel 7 wird bei ca. 120 Grad C mittels einer Handrakel auf Aluminiumfolie (50 µm Dicke) zu 300 µm dicken Schichten ausgezogen und nach dem Erkalten ausgestanzt. Jede Einzeldosis enthält 45 mg Ketoprofen.

Die Bestimmung der Wirkstoffabgabe gemäß Beispiel 7 ergab folgende Werte:
1 Std.: 31,3 %
3 Std.: 66,6 %
6 Std.: 78,3 %
12 Std.: 88,6 %
18 Std.: 90,3 %
24 Std.: 91,0 %

### Beispiel 9

Eine Mischung aus 166,6 g ®EUDRAGIT L 100 und 166,6 g ®EUDRAGIT RS 100 mit 166,6 g Triethylcitrat wird gemäß Beispiel 7 aufgearbeitet und analysiert. Die Bestimmung der Wirkstoffabgabe in vitro ergab folgende Werte
1 Std.: 2,9 %
3 Std.: 5,7 %
6 Std.: 10,3 %
12 Std.: 18,4 %
18 Std.: 22,8 %
24 Std.: 29,4 %

### Beispiel 10

Die Polymer-Wirkstoffmischung gemäß Beispiel 9 wird entsprechend Beispiel 8 aufgearbeitet und analysiert mit folgenden Resultaten:
1 Std.: 13,6 %
3 Std.: 28,9 %
6 Std.: 40,7 %
12 Std.: 50,0 %
18 Std.: 52,4 %
24 Std.: 53,3 %

### Beispiel 11

Ketoprofenpflaster gemäß Beispiel 9 werden hinsichtlich Wirkstsoffabgabe bei pH 5,5 analysiert in dem Gerät, das im Beispiel 7 beschrieben ist. Die Ergebnisse sind folgendermaßen:
1 Std.: 1,6 %
3 Std.: 1,9 %
6 Std.: 2,5 %
12 Std.: 3,1 %
18 Std.: 3,9 %
24 Std.: 5,2 %

### Beispiel 12

Ketoprofenpflaster gemäß Beispiel 10 werden nach Beispiel 11 bei pH 5,5 analysiert. Die Wirkstoffabgabe erfolgt schneller:
1 Std.: 7,1 %
3 Std.: 9,6 %
6 Std.: 12,5 %
12 Std.: 16,1 %
18 Std.: 19,1 %
24 Std.: 21,8 %

## Patentansprüche

1. Schichtförmiges dermales therapeutisches System mit verzögerter Wirkstoffabgabe mit mindestens einem pharmazeutischen Wirkstoff,
**dadurch gekennzeichnet,**
**daß** eine oder mehrere Schichten aus Mischungen von Poly(meth)acrylaten aufgebaut sind und aus einer Schmelze hergestellt werden und die Mischungskomponente 1 aus (Meth)acryl-Polymeren besteht, die Copolymerisate von Methacrylestern und Acrylestern und gegebenenfalls auch weiteren Vinylmonomeren, mit Monomeren **mit funktionellen Gruppen sind, wodurch die Polymere** kationischen, anionischen oder hydrophilen **Charakter haben** enthalten, **und** die Mischungskomponente 2 Polymere sind, die im wesentlichen aus C₁- bis C₁₂-Alkylestern der (Meth)acrylsäure aufgebaut sind, oder Copolymerisate aus Ethylacrylat und Methylmethacrylat mit ca. 5 % Trimethylammonioethylmethacrylatchlorid sind, die das Fließverhalten regulieren.

2. Dermales therapeutisches System gemäß Anspruch 1, **dadurch gekennzeichnet, daß** die Polymeren der Mischungskomponente 2 Glastemperaturen nach DIN 53445 von -70 bis +80 °C, vorwiegend von +10 bis 70 °C aufweisen.

3. Dermales therapeutisches System gemäß den Ansprüchen 1 bis **2**, **dadurch gekennzeichnet, daß** das Polymer-Wirkstoff-System noch niedermolekulare Weichmacher enthält.

4. Verfahren zur Herstellung eines dermalen therapeutischen Systems nach den Ansprüchen 1 bis **3**, durch Vermischen der Feststoffe der Polymerkomponenten, des Wirkstoffs und gegebenenfalls weiterer Zusätze, Erhitzen der Mischung zur Schmelze und Formulierung des therapeutischen Systems aus der geschmolzenen Masse durch deren Verteilung als dünne Schicht, auf dem gegebenenfalls bleibenden Träger oder auf Schutzfolie, die vor der Applikation entfernt wird.

## Claims

1. Layered dermal therapeutic system with delayed release of the active substance, having at least one pharmaceutically active substance, **characterised in that** one or more layers are made up of mixtures of poly(meth)acrylates and are prepared from a melt and component 1 of the mixture consists of (meth)acrylic polymers, which are copolymers of methacrylic esters and acrylic esters and optionally also other vinyl monomers with monomers having functional groups, as a result of which the polymers have a cationic, anionic or hydrophilic nature, and component 2 of the mixture consists of polymers which are essentially made up of C₁-C₁₂-alkyl esters of (meth)acrylic acid, or are copolymers of ethyl acrylate and methyl methacrylate with about 5% trimethylammonioethyl methacrylate chloride which regulate the flow properties.

2. Dermal therapeutic system according to claim 1, **characterised in that** the polymers of component 2 of the mixture have glass transition temperatures according to DIN 53445 of -70 to +80°C, predominantly from +10 to 70°C.

3. Dermal therapeutic system according to claims 1 to 2, **characterised in that** the system of polymer/active substance also contains low-molecular plasticisers.

4. Process for preparing a dermal therapeutic system according to claims 1 to 3, by mixing the solids of the polymer components, the active substance and optionally other additives, heating the mixture till it melts and formulating the therapeutic system from the molten mass by spreading it out as a thin layer, on any substrate which remains or on a protective film which is removed. before use.

## Revendications

1. Système thérapeutique dermique lamellaire ayant une libération retardée du principe actif, avec au moins un principe actif pharmaceutique,
**caractérisé en ce que**
une ou plusieurs couches sont constituées de poly(méth)acrylates et sont préparées à partir d'un produit de fusion, et le composant de mélange 1 consiste en des polymères (méth)acryliques qui sont des copolymérisats d'esters méthacryliques et d'esters acryliques, et le cas échéant également d'autres monomères vinyliques, avec des monomères ayant des groupes fonctionnels grâce auxquels les polymères ont un caractère cationique, anionique ou hydrophile, et les composants de mélange 2 sont des polymères constitués pour l'essentiel d'esters d'alkyle en C₁ à C₁₂ d'acide (méth)acrylique ou sont des compolymérisats à base d'acrylate d'éthyle et de méthacrylate de méthyle avec environs 5 % de chlorure de méthacrylate de triméthylammonioéthyle qui règlent le comportement à l'écoulement.

2. Système thérapeutique dermique conformément à la revendication 1,
**caractérisé en ce que**
les polymères des composants de mélange 2 possèdent des températures de vitrification selon la norme DIN 53445 allant de -70 à +80° C, d'une manière prépondérationde + 10 à 70° C.

3. Système thérapeutique dermique conformément aux revendications 1 à 22,
**caractérisé en ce que**
le système polymère principe actif renferme encore des agents plastifiants à bas poids moléculaire.

4. Procédé de production d'un système thérapeutique dermique selon les revendications 1 à 3,
**caractérisé par**
mélange des substances solides des composants polymères, du principe actif et essentiellement d'autres additifs, par chauffage du mélange à fusion et par mise en forme du système thérapeutique à partir de la masse fondue, par sa répartition sous forme de couche mince sur le support éventuellement restant ou sur la feuille protectrice qui est éliminée avant l'application.
